# EUROPEAN PATENT SPECIFICATION

(11) **EP 0 529 003 B1**
(45) Date of publication and mention of the grant of the patent: **12.01.1994**
(21) Application number: 91912563.3
(22) Date of filing: 20.06.1991
(51) Int. Cl.: C07K 13/00, C12N 15/31, G01N 33/566, A61K 37/02

(54) **IgG-BINDING PROTEIN**
IG-G BINDENDES PROTEIN
PROTEINE LIANT L'IgG

(30) Priority: 21.06.1990 SE 9002212
(43) Date of publication of application: 03.03.1993
(73) Proprietor: HighTech Receptor AB, 211 20 Malmö (SE)
(72) Inventor: BJORCK, Lars, S-240 17 S. Sandby (SE)
(74) Representative: Fagerlin, Heléne
(86) International application number: SE9100447
(87) International publication number: WO9119740

(56) References cited:
- EP-A- 0 131 142
- EP-A- 0 371 199
- Dialog Information Services, File 154, Medline 85-91, Dialog accession no. 07433319, Akesson P et al: "Proteine H--a novel IgG binding bacterial protein", & Mol Immunol Jun 1990, 27 (6) p523-31
- Dialog Information Services, File 154, Medline 85-91, Dialog accession no. 07330524, Gomi H et al: "The gene sequence and some properies of protein H. A novel IgG-binding protein", & J Immunol May 15 1990, 144 (10) p4046-52

## Description

The present application concerns an IgG-binding protein H. freed from its albumin binding sequence, a DNA-sequence coding for the protein, vectors, host cells containing the DNA, a process for preparing the protein, reagent kit and a pharmaceutical composition.

Protein H is an IgG-binding protein isolated from group A streptococci. (P. Akesson, J. Cooney, F. Kishimoto and L. Björck. 1990. Protein H - a novel IgG-binding bacterial protein. Molec. Immunol. 27 523-531). The molecule has a unique structure (H. Gomi, T. Hozumi, S. Hattori, C. Tagawa. F. Kishimoto and L. Björck. 1990. The gene sequence and some properties of protein H - a novel IgG-binding protein. J. Immunol. 144, 4046-4052) and binds preferentially human IgG. Protein A and protein G, two previously described IgG-binding bacterial proteins, show a much broader IgG-binding capacity, including IgG from most mammalian species. In the case of protein G, the molecule also binds albumin (L. Björck, W. Kastern, G. Lindahl and K. Widebäck. 1987. Streptococcal protein G, expressed by streptococci or by E. coli, has separate binding sites for human albumin and IgG. Molec. Immunol. 24, 1113-1122). In many situations. the higher degree of specificity for human IgG, would make protein H a more valuable tool for binding, detection and purification of human IgG antibodies. For instance, when human monoclonal IgG antibodies are produced, the growth medium for the antibody-producing cells contains fetal calf serum. If these antibodies were to be purified on a column of protein A or protein G Sepharose, such a column would also bind IgG from the calf serum, whereas protein H would bind only human IgG. As mentioned above protein G, the IgG-binding protein of human group C and G streptococci, also show affinity for albumin. Initial experiments demonstrated that radiolabelled protein H did not bind to albumin immobilized on nitrocellulose filters (Akesson et al. 1990 Supra).

However, when protein H was bound to Sepharose, the molecule bound both IgG and albumin. The present invention describes how a non-albumin binding protein H, called protein H', is constructed and produced. Such a modified protein H molecule could become a powerful tool in biotechnology. It is useful for a more specific assay and purification of human IgG, removal or adsorption of excessive IgG from blood and for diagnosis of autoimmune diseases than protein H.

By comparing the binding characteristics and the amino acid sequence of protein H and protein M6 it was indicated that the albumin binding corresponds to the C and D domains of these proteins. The present invention concerns a non-albumin binding protein H which does not contain the C and/or D domains or parts of the D and/or C domains.

The protein according to the invention is capable of binding to the Fc fragment of immunoglobulins and has the following binding specifiicity:
I) It binds strongly to human IgG(IgG1, IgG2, IgG3 and IgG4). human IgGFc and rabbit IgG;
II) It binds weakly to pig IgG;
III) It does not bind to IgGs of mouse, rat, bovine animal, sheep, goat and horse;
IV) It does not bind to human IgGFab, IgA, IgD, IgE and IgM;
V) It does not bind to human serum albumin.

The protein H without albumin binding sequence, according to the invention is a protein with the amino acid sequence of claim 1 from amino acid numer 1 to the end of protein H (amino acid 376 inclusive) minus any fragment within part C1 to the end of part D (from amino acid number 15g to the end of protein H) and subfragments and variants thereof that bind to IgG but not to albumin.

This can be a protein with the amino acid sequence starting at amino acid 1 in claim 1 to amino acid number 283 inclusives i.e. the protein H sequence minus the last part D and subfragments and variants thereof that do not bind to albumin but to IgG;
or a protein with the amino acid sequence of claim 1 from amino acid number 1 to amino acid number 241 inclusive i.e. the protein H sequence minus the parts C3 and D of the protein;
or a protein with the amino acid sequence of claim 1 from amino acid number 1 to amino acid number 199 inclusive i.e. the protein H. sequence minus parts C2, C3 and D of the protein H;
or a protein with the amino acid sequence of claim 1 from amino acid number 1 to amino acid number 158 inclusive i.e parts A and B of protein H.

Enclosed are also subfragments or variants of the protein specifically disclosed in the present application wherein the original amino acid sequence is modified or altered by insertion, addition, substitution, inversion or deletion of one or more.amino acids are within the scope of the present invention as far as they retain the essential binding specificity as mentioned above.

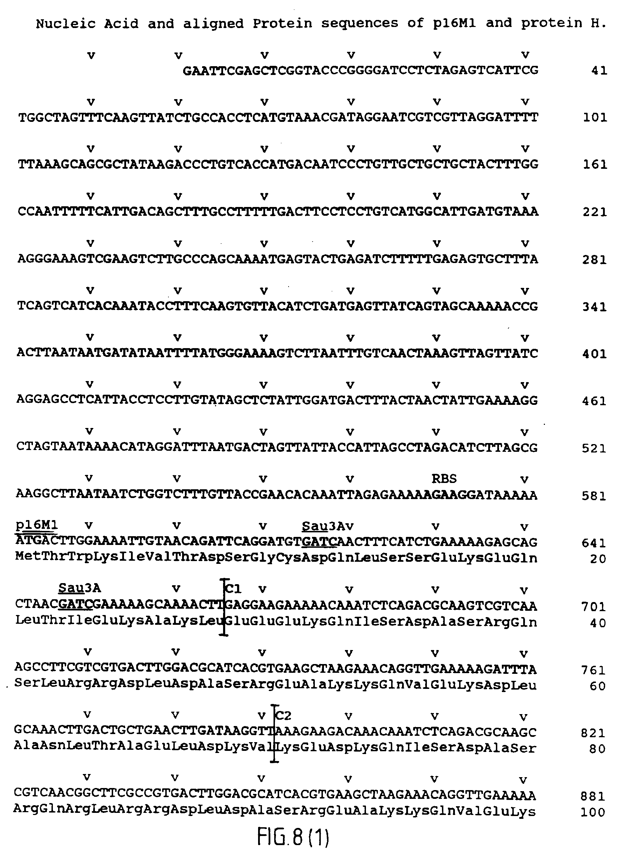

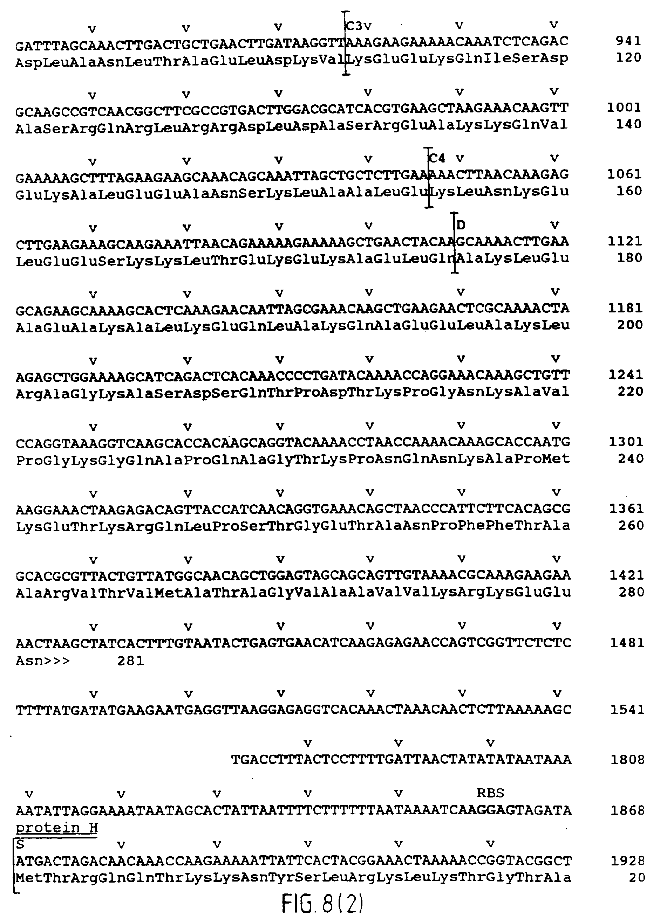

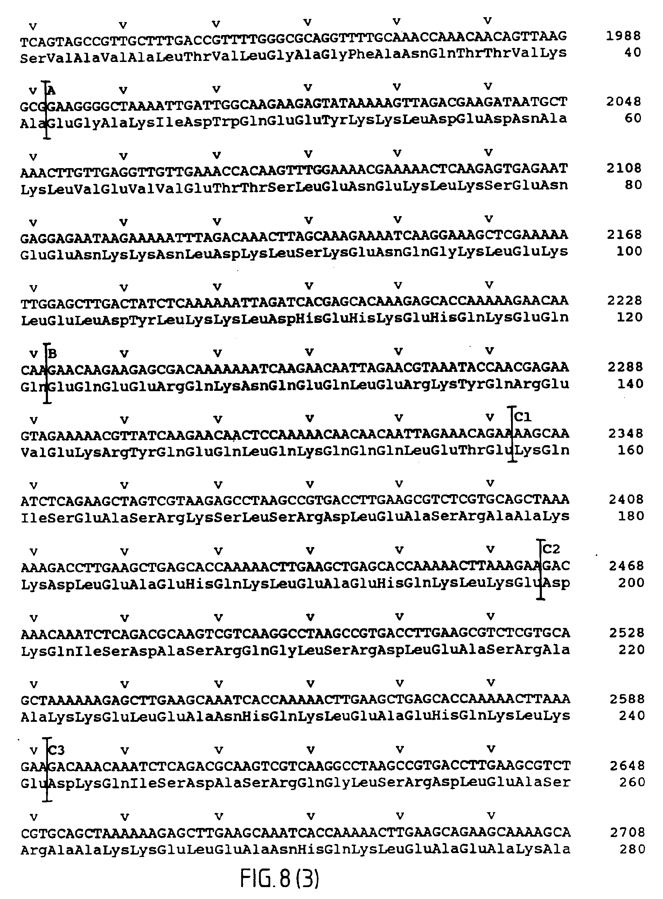

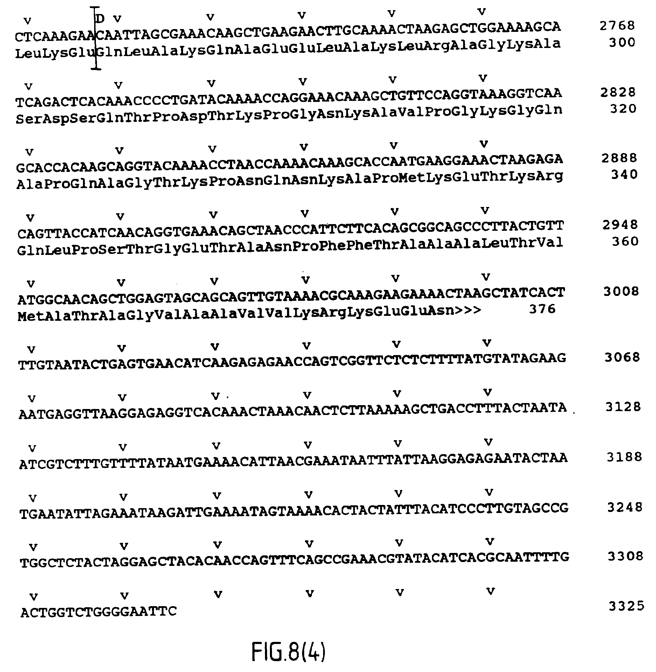

The invention concerns DNA sequences coding for the proteins mentioned above i.e. from base 1868 to base 2342 inclusive of Fig. 8 plus any fragment from base 2342 to base 2996 that do not give the resulting protein the ability to bind to albumin.

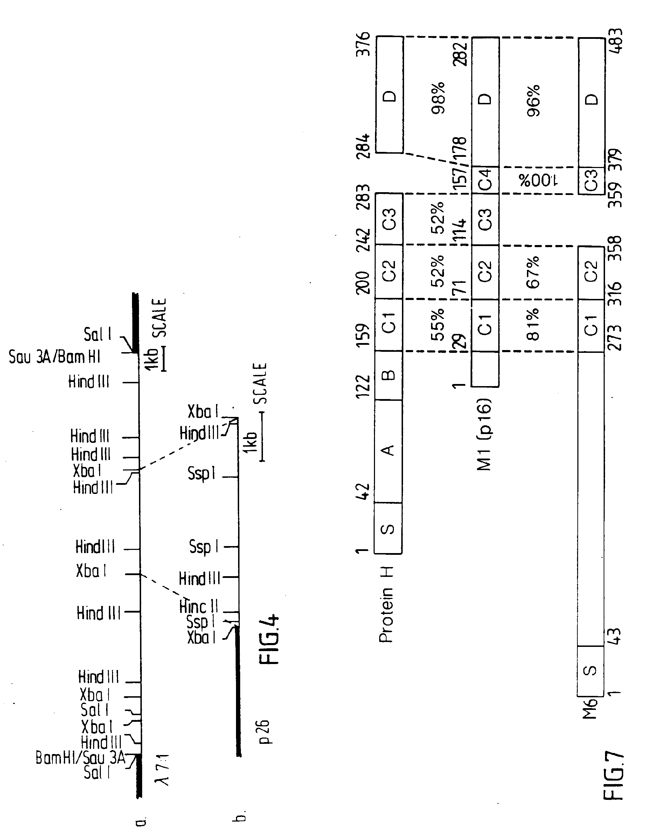

The gene coding for the protein H' can be isolated from the chromosomal DNA of a protein H-producing group A streptococcal strain, such as streptococcus sp. AP1 based on the information in the DNA sequence of the protein H shown in Fig. 8 or from the plasmid p26 (see Fig. 4). The isolation of the gene can also be carried out as follows:

Chromosomal DNA can be isolated from cells of the protein H-producing strain in accordance with known methods (Fahnestock et al, J. Bacteriol. 167, 870(1986). The isolated chromosomal DNA is then segmented into fragments of adequate lengths by biochemical means such as digestion with a suitable restriction enzyme or physical means.

The resulting fragments are then inserted at a suitable restriction site into a suitable cloning vector such as λgt11 (Young et al.. Proc. Natl. Acad.Sci. USA 80, 1194 (1983)) or plasmid vectors such as pUC18 (Messing et al., Gene 33, 103 (1985)).

Alternatively the polymerase chain reaction technique may be used to amplify the 5' part of the protein H gene. Using primers, the region of the protein H gene coding for the signal. A and B or parts of the C and D region can be selectively amplified and cloned. A vector such as pKC30 can be employed. The recombinants are then incorporated into suitable host cells in this case E. coli.

From the resulting transformants, the clones producing the protein which binds to human IgG or the Fc region of human IgG, but not to albumin, are selected by a known method (Fahnestock et al., supra).

After the proteins capable of binding to human IgG or the Fc region of human IgG. but not to albumin, are isolated from the resulting positive clones according to conventional methods, the binding specificities of the proteins are determined to select the clones producing protein H'.

After plasmid DNA of said clone is isolated by conventional methods, the DNA sequence of the insert is determined by known methods (Sanger et al., Proc.Natl. Acad. Sci. USA 74, 5463 (1977): Choen et al., DNA 4, 165 (1985)).

The invention also relates to DNA sequences that hybridize with said identified DNA sequence under conventional conditions and that encode a protein displaying essentially the same binding properties as said protein H. In this context the term "conventional conditions" refers to hybridization conditions where stringent hybridization conditions are preferred.

The expression of genes may be controlled with vectors having the necessary expression control regions in which only the structural gene is inserted. For this purpose, the structural gene shown in Fig. 8 can preferably be used keeping in mind, however, that the whole part C1 to D inclusive must not be present. The structural gene coding for the protein H' can be obtained from the DNA sequence of Fig. 8 or synthesized by conventional methods based on DNA and amino acid sequences given in the present specification.

As for the expression vectors, various host-vector systems have already been developed, from which the most suitable hostvector systems can be selected for the expression of the gene of the present invention.

It has been known that, for each host cell, there is a particularly preferred codon usage for the expression of a given gene. In constructing a gene to be used for a given host-vector system, the codons preferred by the host should be used. Suitable sequences for the gene for the protein H' to be used in a particulate host-vector system can be designed based on the amino acid sequence given in Fig. 8 and synthesized by conventional synthetic methods.

The present invention further relates to a process for producing a truncated form of according to the present application protein H by culturing a host cell transformed with an expression vector into which the gene encoding a truncated form of protein H is inserted.

The process comprises steps of
I) inserting a gene coding for a truncated form of protein H into a vector;
II) introducing the resulting vector into a suitable host cell;
III) culturing the resulting transformant cell to produce a truncated form of protein H; and
IV) recovering the truncated form of protein H from the culture.

In the first step, the gene coding for the truncated form of protein H, which is isolated from the chromosomal DNA of the AP1 streptococcal strain and present in plasmid p26 or synthesized as mentioned above, is inserted into a vector suitable for a host to be used for the expression of the truncated form of protein H. Insertion of the gene can be carried out by digesting the vector with a suitable restriction enzyme and linking thereto the gene by a conventional method, such as ligation.

In the second step, the resulting recombinants plasmid is introduced into host cells. The host cells may be Escherich coli, Bacillus subtilis or Saccharomyces cerevisiae or other cells. The introduction of the expression vector into the host cells can be performed in a conventional way and recombinants expressing IgG binding peptides are then selected.

In the third step, the resulting transformant cells are cultured in a suitable medium to produce as truncated form of protein H by the expression of the gene. The cultivation can be conducted in a conventional manner.

In the fourth step, the produced truncated form of protein H is recovered from the culture and purified, which can be conducted by known methods. For example, the cells are disrupted by known methods such as ultrasonification, enzyme treatment or grinding. The truncated form of protein H released by the cells or secreted into the medium is recovered and purified by conventional methods usually used in the field of biochemistry such as ion-exchange chromatography, gel filtration, affinity chromatography using IgG as ligand, hydrophobic chromatography or reversed phase chromatography, which may be used alone or in suitable combinations.

As mentioned above, the protein provided by the present invention can be used for identification or separation of human IgG. For these purposes, the protein may be brought into a reagent kit or pharmaceutical composition by combining or mixing it with suitable reagents, additives or carriers. When used for treating human blood these additives should be pharmaceutically acceptable. As carriers there can be used Sepharose® activated with CNBr, polyacrylamide beads and gold particles.

The following figures are attached:

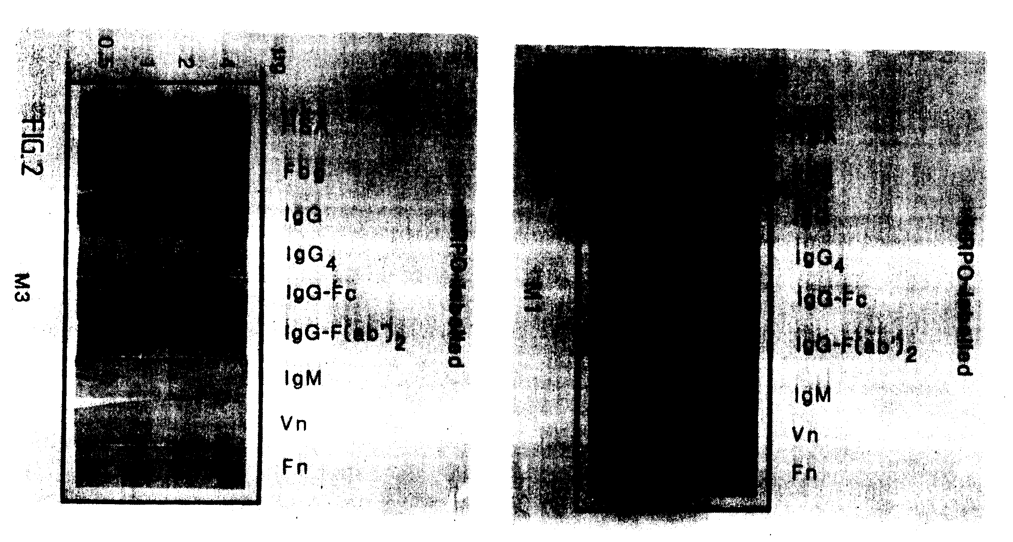

Figure 1: Dot binding to M1 protein (isolated from culture supernatants) applied to strips of nitrocellulose in different amounts. The strips were probed with HRPO-labelled proteins. Human serum albumin (HSA), fibrinogen (Fbg), human polyclonal immunoglobulin G (IgG), a monoclonal human IgG of subclass 4 (IgG4) and Fc fragments of human IgG (IgG-Fc) all bound to M1 protein, whereas F(ab')₂ fragments of IgG (IgG-F(ab')₂), human polyclonal immunoglobulin M (IgM), vitronectin (Vn) and fibronectin (Fn) did not bind.

Figure 2: Dot binding to M3 protein (extracted from strain 4/55 streptococci with C-phage associated lysin) applied to strips of nitrocellulose in different concentrations. The strips were probed with HRPO-labelled proteins (for explanation of abbreviations see legend to Fig. 1). The M3 protein was found to bind human serun albumin (HSA) and fibrinogen (Fbg) strongly, and showed weak affinity for human polyclonal IgG(IgG) and fibronectin (Fn).

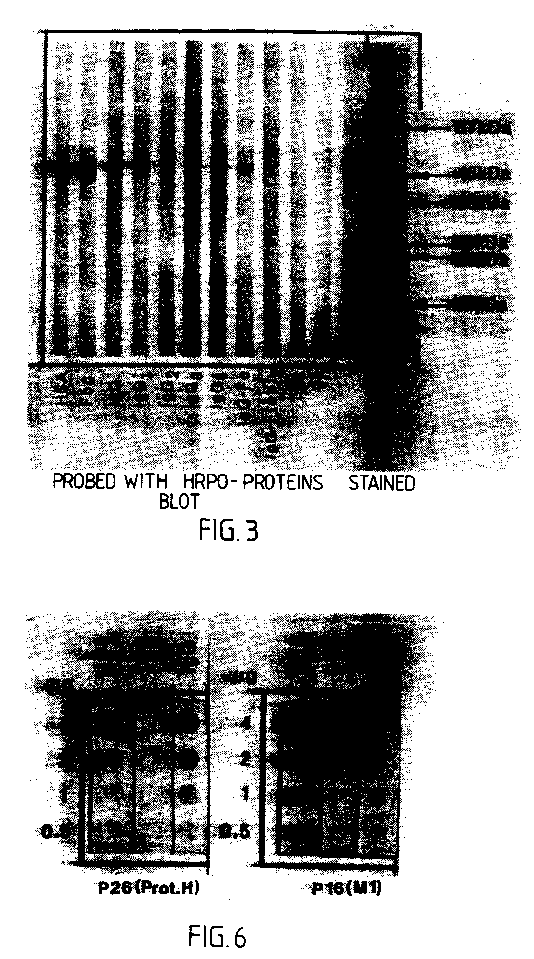

Figure 3: SDS-polyacrylamide electrophoresis (SDS-PAGE) in a 10% gel of 100 µg samples of extracellular M1 protein was followed by transfer of the separated protein samples to strips of nitrocellulose. Strips were then probed with different HRPO-labelled proteins. The two right lanes show (after staining with amidoblack) the separation of M1 protein and molecular weight markers on 10% SDS-PAGE.

The figure shows binding of albumin (HSA), fibrinogen (FbG), polyclonal IgG (IgG), monoclonal IgG's(IgG1 - IgG4) and Fc fragments of IgG(IgG-Fc) to M1 protein.

Figure 4: Restriction maps of lambda EMBL3 clone 7:1 (a) and plasmid pK 18 clone p26 (b). Clone p16 is the indicated SspI fragment cloned into pUC18.

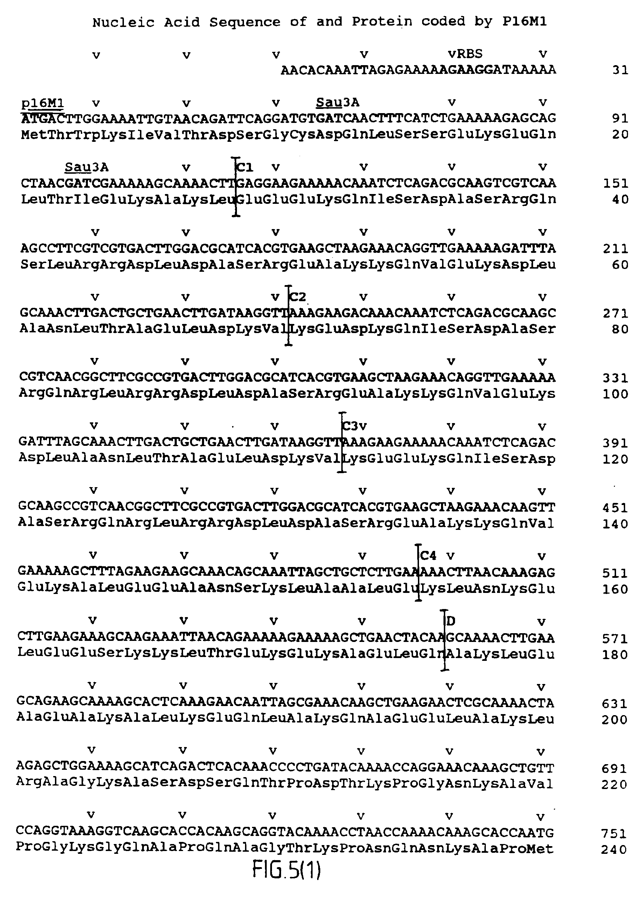

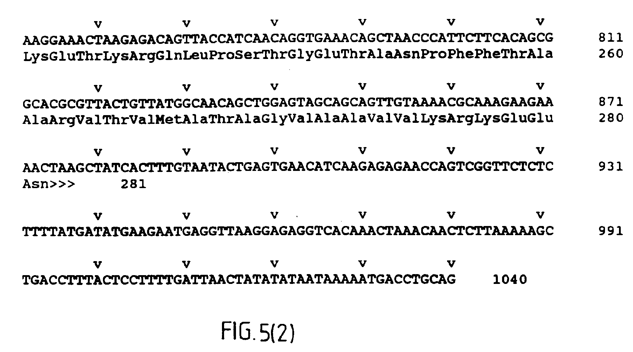

Figure 5: Nucleic acid and protein sequence of the albumin-binding fragment of streptococcal M protein type 1 encoded by p16.

Figure 6: p16 encoded fragment of M1 protein and p26 encoded protein H were applied separately to strips of nitrocellulose. The strips were then incubated with HRPO-labelled human serum albumin (HSA), human fibrinogen or polyclonal human IgG (IgG). The figure shows binding of HSA to the fragment of M1 and to protein H. Protein H also binds IgG.

Figure 7: Comparison of homologous regions in protein H, a fragment of M protein type 1 (p16) and M protein type 6 (M6). The amino acid sequence of encoded p16 M1 was compared to M6 and protein H, and the homology is indicated as percentage identical amino acids. The three sequences were aligned to achieve maximal homology. All three peptides bind albumin, which maps the albumin-binding activity to the C and/or D regions.

Figure 8: Nucleic acid sequence of the region of p26 involved in encoding the albumin binding portion of M protein type 1 and protein H. The corresponding amino acid sequences are given.

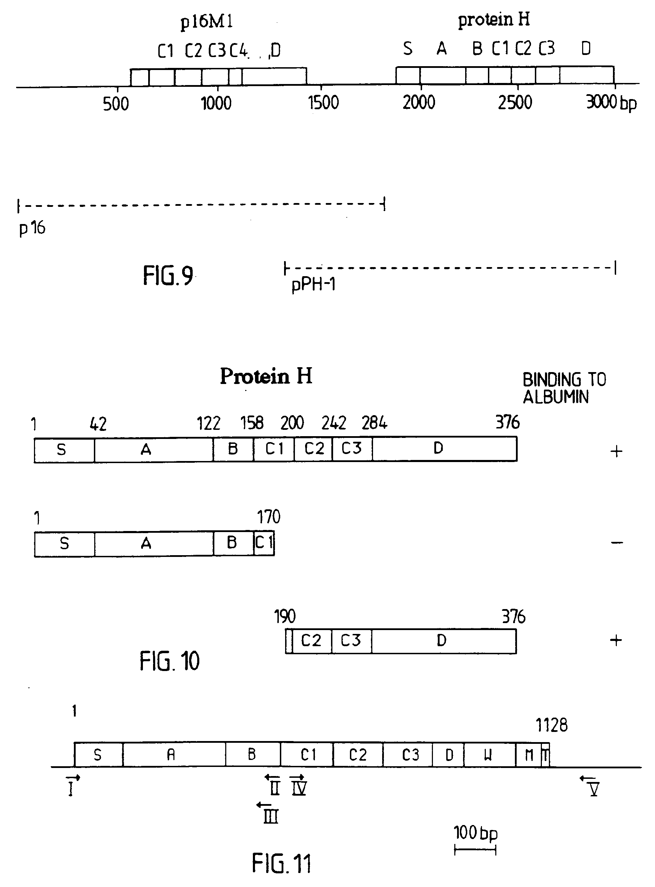

Figure 9: Physical organization of the genes for p16M1 and protein H. The thin line represents the known DNA sequences. The open reading frames corresponding to p16M1 and protein H genes are indicated on this line. The division of the genes into subregions is indicated. The base coordinates are under the line whereas the figures above the line are the amino acid coordinates. The sequences corresponding to the insert DNA in P16 and pPH-1 are represented, respectively, by the two hirozontal broken lines.

Figure 10: Schematic representation of protein H and two sub-fragments and the ability of the corresponding polypeptides to bind albumin. Amino acid residue numbers are indicated and so are the subregions of the peptides.

Figure 11: Schematic representation of the cloned protein H gene. Indicated are S, signal sequence; A and B unique regions; C1, C2, C3 repeated sequence; D, D region; W, wall spanning region; M membrane spanning region; T, charged C-terminal residues. Horizontal arrows labelled I, II, III, IV and V indicate the position of the primers.

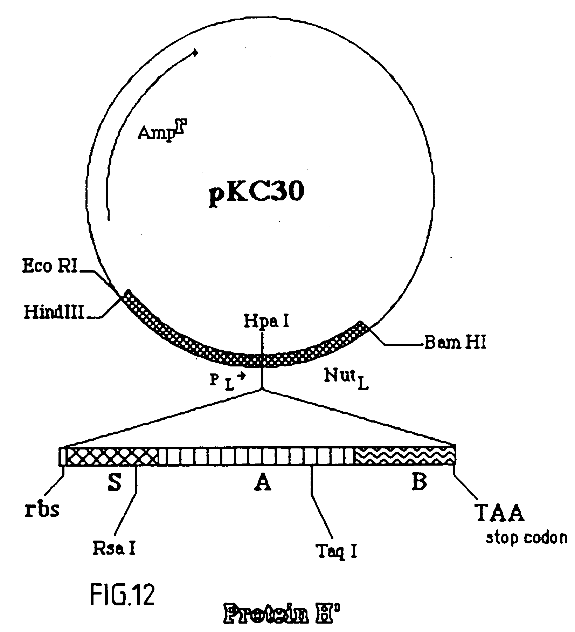

Figure 12: Shows a map of the truncated protein H gene (protein H') in the expression vector pKC30. The ribosome binding site (rbs) is indicated and several important restriction sites.

The present invention will more precisely be described by the following examples. But, they are not intended to limit the scope of the present invention.

### Example 1

Isolation of albumin-binding proteins from Streptococcus pyogenes.

Strains of Streptococcus pyogenes M type 1 (40/58) and M 3 (4/55) of the strain collection of the WHO Streptococcal Reference Laboratory, Prague, Czechoslovakia were grown in 5 l of Todd Hewitt Broth (Difco) by using a 7 l fermentor. The culture was inoculated with 500 ml of an over night stand culture and fermenation was performed for 18 hours at 37°C at pH 7.2 and with regulation of the glucose concentration at 0.2%. The culture supernatants and the cell pellets were saved separately and used for purification of albumin-binding proteins.

### 1. Preparation of albumin-binding proteins from culture supernatants

Proteins in the culture supernatants were precipitated with ammonium sulphate at 70% saturation. The precipitate was dialyzed in the presence of 50 mM phenylmethylsulfonyl fluoride against distilled water and finally against 50 mM sodium acetate, pH 4.5. This crude preparation was applied to 200 ml of CM-Sepharose C1 4B equilibrated with 50 mM Na-acetate, pH 4.5, and washed with the same buffer. The column was then rinsed with 200 mM sodium phosphate buffer. pH 6.0. Fractions showing binding activity to fibrinogen and human serum albumin (HSA) in dot binding to nitrocellulose filter were collected and directly applied to affinity chromatography on fibrinogen-Sepharose (50 ml). The column was washed with PBS and bound proteins were eluted with 2 M KSCN buffer. Fractions which were positive in dot binding on nitrocellulose against fibrinogen and albumin were collected, dialyzed against 50 mM ammonium hydrogen carbonate and applied to albumin-Sepharose (20 ml). The column was washed and eluted as described above and fractions eluted with 2 M KSCN buffer, which showed fibrinogenand albumin-binding were collected and dialyzed against 50 mM ammonium hydrogen carbonate. The albumin- and fibrinogen-binding material was stored in alliquots or lyophized.

### 2. Preparation of albumin-binding protein from cell pellets obtained with C-phage associated lysin.

Streptococcal cell pellets of the two strains (about 60 g of bacteria wet weight) were each suspended in 300 ml PBS containing 10 mg Penicillin and 1 ml 2-mercaptoethanol. Then 10 ml of phage lysin prepared according to Cohen et al. (Applied Microbiol. 29, 175-178, 1975) was added and cells incubated for 6 hours at 37°C with stirring. The resulting lysate was centrifuged and the pellet washed with 100 ml of PBS. The resulting supernatants were pooled , dialyzed against water containing 50 mM sodium acetate, pH 4.5, and fractionated on CM-Sepharose as described above. Albumin- and fibrinogen-binding fractions were further purified on fibrinogenand albumin-Sepharose, also as described above.

### Example 2

Characterization of the isolated albumin-binding streptococcal proteins

Streptococcal M proteins (for a review on M protein see Fischetti 1989. Clin. Microbiol. Rev. 2, 285) are known to bind fibrinogen and the albumin-binding proteins from both streptococcal strains (M protein type 1 and type 3) did indeed show affinity for both fibrinogen and albumin (purified first on fibrinogen-Sepharose and then on albumin-Sepharose). Further analysis of the proteins demonstrated serological identity with M1 and M3 protein. respectively. Thus, it was concluded that the albumin-binding proteins of the two strains represented M1 and M3 proteins.

On SDS-polyacrylamide electrophoresis (SDS-PAGE) the estimated molecular weight of M1 was 49 kDa, whereas the M3 protein was more size heterogeneous with several bands around 60 kDa. In Figs. 1 and 2, M1 and M3 proteins were applied to nitrocellulose filters and probed with some human plasma proteins labelled with horseradish peroxidase (HRPO) as described by Tijssen (In: Laboratory techniques in Biochemistry and Molecular Biology. Eds. Burdon and van Knippenberg. Elsevier, Amsterdam-New York-Oxford 1985). Both M proteins bound albumin. HRPO-labelled M1 and M3 proteins were also both found to react with albumin applied to nitrocellulose filters. As shown in figure 1, M1 protein also showed affinity for HRPO-labelled fibrinogen and immunoglobulin G (IgG). In the case of IgG the interaction was mediated through the Fc region. M3 protein showed a much weaker affinity for IgG but reacted strongly with HRPO-labelled albumin and fibrinogen (figure 2). The binding properties of M1 protein was also studied by Western blot analysis. As shown in Fig. 3, M1 separated by SDS-PAGE and blotted onto nitrocellulose filters, reacted with albumin, fibrinogen and IgG.

### Example 3

Identification and characterization of the albumin-binding part of M proteins and protein H.

A genomic library of the streptococcus M type 1 strain 40/58 was made in the lambda replacement vector λ EMBL3. The DNA was partially cleaved with the restriction enzyme Sau3A and ligated, without size fractionation, to vector DNA cleaved with Bam HI. Recombinants expressing M protein type 1 (M1) were detected using specific anti-M1 serum. Three reacting clones were selected for further study. Fig. 4 is a physical map on one of these clones, named λ7:1.A 4.4 kb Xba I fragment of lambda 7:1 was ligated to the plasmid vector pK18, and the ligation mix used to transform E.coli TB1. Transformants harbouring the correct plasmid were initially selected using plasmid screening and confirmed by restriction enzyme digest analysis. One strain carrying the correct plasmid, named p26 was selected for further study. p26 (Fig. 4b) was shown to contain the entire gene sequence for protein H an IgG binding protein of streptococcus, and a truncated M1 gene sequence (Akesson et al. 1990 supra; Gomi et al.1990 supra). p16 was generated by subcloning the 1.6 kb Ssp I fragment of p26. This fragment is indicated in Fig. 4b, and is upstream of the protein H gene which is located on the smaller Ssp I fragment. The entire insert of p16 was sequenced and within this sequence an open reading frame corresponding to 281 amino acids was identified (Fig. 5).

Lysates of E. coli harbouring either p26 or p16 were applied to IgG-Sepharose and albumin-Sepharose, respectively. Proteins bound were eluted with glycine buffer pH2.0 and 0.15 to 0.63 mg of IgG or albumin bindning proteins were isolated per 1 of culture of the p26 and p16 clones, respectively. The N-terminal amino acid sequences of p26 encoded protein H and p16 encoded M1 were determined (10 amino acids each) and found to be fully compatible with the gene sequences of protein H (Gomi et al.1990 supra) and the truncated M1 sequence shown in Fig. 5. Eluted proteins were also analyzed in binding experiments. As expected protein H (from the p26 lysate) reacted with HRPO-labelled human IgG. However, also HRPO-labelled albumin reacted with this IgG-binding protein (Fig. 6), whereas the protein did not react with albumin when it was labelled with 125-I using lactoperoxidase or chloramin-T, suggesting that this labelling procedure destroys the albumin-binding activity of protein H.

The albumin-binding property of p26 encoded protein H was further demonstrated when the protein was coupled to Sepharose. Thus, p26 encoded protein H coupled to Sepharose bound albumin (and IgG) very effectively. Finally, protein H did not bind fibrinogen (Fig. 6). The fragment of M1 protein expressed by p16. also showed affinity for albumin. Fibrinogen-binding was lost in the p16 encoded M1 fragment as compared to entire M1 (figure 6). Also IgG-binding seemed to be lost, but additional experiments showed that in solution p16 M1 could block the binding of entire M1 protein to IgG, suggesting that part of the IgG-binding site is present on the p16 M1 protein fragment. To summarize: protein H and M proteins (exemplified with M type 1 and 3 proteins) all show affinity for albumin and IgG whereas M proteins, but not protein H. also bind fibrinogen.

The amino acid sequence of p16 M1 was then compared to the sequences of protein H (Gomi et al. 1990 supra) and M protein type 6 (Hollingshead et al. 1986. J. Biol. Chem. 261 1677).

As shown in Fig. 7 there is a striking homology between the p16 encoded M1 sequence and the C-terminal sequences of protein H and M6, indicating that the albumin-binding region corresponds to the C and D domains of these proteins. Thus, any protein sequence homologous to the sequence encoded by p16 is likely to bind albumin. It is also interesting to note that M1 and M3. proteins bind IgG. Fibrinogen-binding is a well-known property of M proteins which was also demonstrated for the M1 and M3 protein used in this study, whereas protein H did not bind fibrinogen.

### CONCLUSIONS:

1. Proteins showing homology with the p16 sequence of Fig. 5 will most probably bind albumin. In the present work this was exemplified with the binding of albumin to M proteins 1 and 3, a fragment of M1 corresponding to the p16 sequence of Fig. 5. and protein H.
2. M proteins type 1 and 3 were also found to bind IgG and at least in the case of M1 this binding was mediated through the Fc region.

### Example 4

Production of a form of protein H which binds IgG but does not bind albumin.

However, a form of protein H which no longer had the ability to bind albumin was generated by deletion of the C-terminal half of the gene. As demonstrated above albumin binding of protein H is located to C and/or D-regions.

Using polymerase chain reaction (PCR) the 5' part of protein H gene was amplified. The PCR technique is an in vitro method in which genomic or cloned target sequences are specifically enzymatically amplified as directed by a pair of oligonucleotide primers. (J.F. Williams. 1989. optimization strategies for the polymerase chain reaction. Biotechniques 7: 762-768).Thus, the region of the protein H gene coding for the signal and the A and B region can be selectively amplified and cloned.

Using primers I (CAAGGAGTAGATAATGACTAG) and II (TTATGTTTCTAATTGTTGTTGTTTTTGGAGTTG), whose positions are indicated on the map of protein H gene (Fig. 11). this region was obtained. As primer II contains a low G+C content (9/33 bases) and a run of T residues, it is not an ideal primer (according to the criteria defined by Williams, 1989). Sacrificing the last 7 amino acid residues of the translated B region a possible primer III (TTATTGGAGTTGTTCTTGATAACG) can be used. This has 8/24 G+C pairs and no run of any single residue.

Both primers II and III have been designed to incorporate sequence which results in an inframe translational stop codon immediately 3' to the amplified fragment to prevent read-through into vector sequences. Primer I contains the ribosome binding site of the protein H gene.

0.25 ng of p26 plasmid DNA was used as the target for the PCR. The primers were added to a final concentration of 0.1 µM by dilution from a 1 µM stock. The reaction buffer contained 250 µM dATP, dCTP, dGTP and dTTP in 10 mM Tris-HCl pH 8.3. 50 mM KCl, 1.5 mM MgCl₂ 0.001% gelatine. 100 µl of mineral oil was layered over the reaction mix. The DNA was denatured by heating to 98°C and then cooled to 53°C after which 2 units of Taq polymerase enzyme was added.

30 cycles of the following steps were performed.
Step 1:
1 min at 92°C,
Step 2:
1 min at 53°C,
Step 3:
2 min at 72°C.
A Cetus intellegent heating block, set on mode 4, was used to control the temperature changes.

After completion of the 30 cycles the sample was cooled to room temperature A 10 µl aliquot of the reaction mixture was electrophoresed on a 3% agarose gel. This sample contained 500 ng of a DNA fragment of either 487 bp or 465 bp depending on whether primers I and II or I and III respectively were used. As there was only one DNA product of this reaction the remaining sample could be used directly for further steps without size fractionation.

The reaction mix was deproteinized by extraction with phenol saturated with 10 mM Tris-HCl pH 8.0, 1 mM EDTA. The extracted material was treated with cholorform-isoamylalcohol (mixed in a 24:1 ratio and saturated with water) to remove excess phenol. The DNA was then precipitated by addition of a 1/10 volume of 8 M LiC1 and 2.5 volumes of ice-cold ethanol. The recovered DNA was dried under vacuum in a Speedy-Vac and resuspended in water to a final concentration of 200 ng/µl 400 ng of product was ligated to 200 ng of pKC30 (M. Rosenberg, Y.S. Ho and A. Shatzman. 1989. The use of pKC30 and its derivatives for controlled expression of genes. pp, 429-444 In R. Wu, L. Grossman and K. Moldave (eds.). Recombinant DNA methodology. Academic Press new York) which had been previously cleaved with HpaI deproteinized and precipitated as described for the PCR product. The molar ratio of insert to vector was approx. 5:1. The ligation was performed using T4 ligase (Gibro BRL) and the buffering system supplied in accordance with the manufacturer's instructions.

Samples of the ligation mix corresponding to 25, 50, 75 and 100 nq of vector DNA were transformed into E. coli strain C600 cells lysogenized with λ were made competent using the RbCl/CaCl₂ method of Kushner (1978). This strain does not allow expression from the P1 of pKC30. Colony hybridization experiments (Grundstein and Hogness, 1975) were performed on the resulting transformants using the generated PCR fragment as a probe. 10 colonies which appeared to hybridize were selected and plasmid DNA prepared from these according to the method of Birnboim and Doly (1979). The DNA was further purified by treatment with RNase, deproteinized and precipitated. Further hybridization studies and restriction enzyme digest analysis of the plasmids confirmed the presence of the desired DNA fragment. The correct orientation of the insert with respect to the P1 promoter was established for several of the plasmids.

The presence of the correct fragment was confirmed by the ability of the purified plasmids to hybridize in Southern blots to digoxiginin labelled amplified fragment, using Boehringer-Mannheim's non-radio active DNA labelling kit and by digestion of the plasmids with the restriction enzymes Taq I - RsaI in a double digest. This produced a characteristic 240 bp fragment which also hybridized to the labelled PCR product in Southern blots.

The orientation of the PCR fragment with respect to the promoter was confirmed by the presence of a 1.9 Kb Hind III-RsaI fragment which is replaced by a 2.1 Kb fragment when the cloned fragment is in the opposite orientation to the promotor. See Fig 12 which shows a map of the truncated protein H gene (protein H') in the expression vector pKC30. The ribosome binding site (rbs) is indicated and several important restriction sites.

One of the plasmids was selected for further studies and was called pPH465. This plasmid was transformed into E. coli strain pop 2136 as described above. Simple plasmid screening established that the intact plasmid had been transformed. The strain harbouring pPH465 was called PH465. Strain PH465 was grown in liquid culture at 32°C until the A₆₀₀was 0.6. Expression of protein H' was induced by increasing the culture temperature to 42° for 20 min and growth was continued at 37° for 2 hr. The cells were collected by centrifugation and protein H' was purified from cell lysates and the culture supernatent as outlined below.

If primers I and II were employed a 487 base pair fragment was amplified. The corresponding polypeptide was 157 amino acid residues long (Mr 20993). If primers I and III were used a 465 base pair fragment resulted with a polypeptide of 150 amino acids (Mr 20112) being encoded. The peptides did not bind to albumin.

The promoter incorporated in pKC30 is the lambda P1 promotor. A complete description of the construction of pKC30 and its uses are given in Rosenberg et al. 1989 supra. This promoter was found to be 8-10 times more efficient than Plac. This high activity has been shown to detrimental in some cases and can lead to plasmid instability. By choosing a bacterial host which contains an integrated copy of lambda. the expression from P1 can be repressed completely by lambda encoded cI (repressor protein) which increases stability. The protein H' (the truncated gene) clone was selected in this background by colony hybridization using digoxiginin labelled amplified fragment as probe and plasmid DNA was prepared. This DNA was used to transform a host which is lysogenized with lambda carrying a temperature - sensitive mutation in cI (cI857) where expression of the protein H gene fragment from the P1 promotor can be regulated.

Cultures of cells harbouring pKC30/protein H' (the truncated gene) were first grown at 32°C so high cell density was reached without expression from P1. Expression was then induced by increasing the temperature to 42°C. Expression was rapid - which is beneficial as the protein H fragment is lethal and is degraded rapidly.

### Purification of the protein H'

The E. coli cells from a 5 l culture were lysed and the resulting lysate was subjected to affinity chromatography on IgG-Sepharose. Bound protein was eluted with glycine buffer pH 2.0, and a single protein band with a molecular weight of 22 kDA on SDS-PAGE was obtained. In total the amount of purified protein was 0.3-0.7 mg/l culture. In Western blots this material bound radiolabelled human lgG but not human radiolabelled albumin. Moreover, when the protein H' peptide was radio labelled it bound to IgG-Sepharose but not to albumin-Sepharose. Finally, the radiolabelled peptide did not form complexes with albumin in solution, as judged by immunoprecipitation experiments with goat anti-human albumin antibodies. Taken together, these data demonstrate that the protein H' peptide represents an IgG-binding fragment of the entire protein H molecule devoid of ilbumin-binding activity.

## Claims

1. A truncated form of protein H incapable of binding to albumin, characterized in that any fragment from amino acid number 159 to amino acid number 376 of the amino acid sequence of protein H reported below is deleted, and subfragments and variants of the said truncated form of protein H that do not bind albumin, wherein in said variants the original amino acid sequence is modified or altered by insertion, addition, substitution, inversion or deletion of one or more amino acids.

MetThrArgGlnGlnThrLysLysAsnTyrSerLeuArgLysLeuLysThrGlyThrAla 20

SerValAlaValAlaLeuThrValLeuGlyAlaGlyPheAlaAsnGlnThrThrValLys 40

A
AlaGluGlyAlaLysIleAspTrpGlnGluGluTyrLysLysLeuAspGluAspAsnAla 60

LysLeuValGluValValGluThrThrSerLeuGluAsnGluLysLeuLysSerGluAsn 80

GluGluAsnLysLysAsnLeuAspLysLeuSerLysGluAsnGlnGlyLysLeuGluLys 100

LeuGluLeuAspTyrLeuLysLysLeuAspHisGluHisLysGluHisGlnLysGluGln 120

B
GlnGluGlnGluGluArgGlnLysAsnGlnGluGlnLeuGluArgLysTyrGlnArgGlu 140

C1
ValGluLysArgTyrGlnGluGlnLeuGlnLysGlnGlnLeuGluThrGluLysGln 160

IleSerGluAlaSerArgLysSerLeuSerArgAspLeuGluAlaSerArgAlaAlaLys 180

C2
LysAspLeuGluAlaGluHisGlnLysLeuGluAlaGluHisGlnLysLeuLysGluAsp 200

LysGlnIleSerAspAlaSerArgGlnGlyLeuSerArgAspLeuGluAlaSerArgAla 220

AlaLysLysGluLeuGluAlaAsnHisGlnLysLeuGluAlaGluHisGlnLysLeuLys 240

C3
GluAspLysGlnIleSerAspAlaSerArgGlnGlyLeuSerArgAspLeuGluAlaSer 260

v v v v
ArgAlaAlaLysLysGluLeuGluAlaAsnHisGlnLysLeuGluAlaGluAlaLysAla 280
D
v v v v
LeuLysGluGlnLeuAlaLysGlnAlaGluGluLeuAlaLysLeuArgAlaGlyLysAla 300
v v v v
SerAspSerGlnThrProAspThrLysProGlyAsnLysAlaValProGlyLysGlyGln 320
v v v v
AlaProGlnAlaGlyThrLysProAsnGlnAsnLysAlaProMetLysGluThrLysArg 340
v
GlnLeuProSerThrGlyGluThrAlaAsnProPhePheThrAlaAlaAlaLeuThrVal 360
v v v v
MetAlaThrAlaGlyValAlaAlaValValLysArgLysGluGluAsn 376

2. A protein according to claim 1, characterized in that it consists of the amino acid sequence from amino acid number 1 to number 158 inclusive, which protein is called protein H'.

3. A DNA sequence coding for the protein according to any of the Claims 1 or 2, as it can be deduced from the sequence below.

ATGACTAGACAACAAACCAAGAAAAATTATTCACTACGGAAACTAAAAACCGGTACGGCT 60
v v v v v v
TCAGTAGCCGTTGCTTTGACCGTTTTGGGCGCAGGTTTTGCAAACCAAACAACAGTTAAG 120
v v v v v v
GCGGAAGGGGCTAAAATTGATTGGCAAGAAGAGTATAAAAAGTTAGACGAAGATAATGCT 180
v v v v v v
AAACTTGTTGAGGTTGTTGAAACCACAAGTTTGGAAAACGAAAAACTCAAGAGTGAGAAT 240
v v v v v v
GAGGAGAATAAGAAAAATTTAGACAAACTTAGCAAAGAAAATCAAGGAAAGCTCGAAAAA 300
v v v v v v
TTGGAGCTTGACTATCTCAAAAAATTAGATCACGAGCACAAAGAGCACCAAAAAGAACAA 360
v v v v v v
CAAGAACAAGAAGAGCGACAAAAAAATCAAGAACAATTAGAACGTAAATACCAACGAGAA 420
v v v v v v
GTAGAAAAACGTTATCAAGAACAACTCCAAAAACAACAACAATTAGAAACAGAAAAGCAA 480
v v v v v v
ATCTCAGAAGCTAGTCGTAAGAGCCTAAGCCGTGACCTTGAAGCGTCTCGTGCAGCTAAA 540

v v v v v v
AAAGACCTTGAAGCTGAGCACCAAAAACTTGAAGCTGAGCACCAAAAACTTAAAGAAGAC 600

v v v v v v
AAACAAATCTCAGACGCAAGTCGTCAAGGCCTAAGCCGTGACCTTGAAGCGTCTCGTGCA 660

v v v v v v
GCTAAAAAAGAGCTTGAAGCAAATCACCAAAAACTTGAAGCTGAGCACCAAAAACTTAAA 720

v v v v v v
GAAGACAAACAAATCTCAGACGCAAGTCGTCAAGGCCTAAGCCGTGACCTTGAAGCGTCT 780

v v v v v v
CGTGCAGCTAAAAAAGAGCTTGAAGCAAATCACCAAAAACTTGAAGCAGAAGCAAAAGCA 840

v v v v v v
CTCAAAGAACAATTAGCGAAACAAGCTGAAGAACTTGCAAAACTAAGAGCTGGAAAAGCA 900

v v v v v v
TCAGACTCACAAACCCCTGATACAAAACCAGGAAACAAAGCTGTTCCAGGTAAAGGTCAA 960

v v v v v v
GCACCACAAGCAGGTACAAAACCTAACCAAAACAAAGCACCAATGAAGGAAACTAAGAGA 1020

v v v v v v
CAGTTACCATCAACAGGTGAAACAGCTAACCCATTCTTCACAGCGGCAGCCCTTACTGTT 1080

v v v v v .
ATGGCAACAGCTGGAGTAGCAGCAGTTGTAAAACGCAAAGAAGAAAACTAA 1131

4. A DNA sequence hybridizing to a DNA sequence of Claim 3. under conventional conditions and encoding a protein displaying the same binding properties as the proteins of any one of Claims 1 or 2.

5. A recombinant plasmid vector containing a DNA sequence of any one of Claims 3 or 4.

6. A host cell transformed with the recombinant plasmid of Claim 5.

7. A host cell according to Claim 6, which belongs to the species E. coli.

8. A process for producing a form of protein H according to Claims 1 or 2, comprising cultivating a host cell according to Claim 6 or 7 under suitable conditions, accumulating said protein in the culture or lysing the cells and recovering it thereform.

9. A reagent kit for binding, separation and identification of human immunoglobulin G, characterized in that it comprises a protein according to any one of Claims 1 or 2.

10. A composition comprising a protein according to any of Claims 1 or 2 and optionally additives or carriers.

11. A pharmaceutical composition comprising a protein according to any of claims 1 or 2 and optionally pharmaceutical acceptable additives or carriers.

## Patentansprüche

1. Verkürzte Form eines Protein H, unfähig sich an Albumin zu binden, dadurch gekennzeichnet, daβ irgendein Fragment von Aminosäure-Nr. 159 bis Aminosäure-Nr. 376 der Aminosäurefrequenz des Protein H, wie unten dargestellt, entfernt ist, und Subfragmente und Varianten dieser verkürzten Form des Protein H, das nicht Albumin bindet, wobei in besagten Varianten die Originalaminosäurefrequenz modifiziert oder verändert ist durch Einfügung, Addition, Substitution, Inversion oder Entfernung von einer oder mehreren Aminosäuren.

v v
MetThrArgGlnGlnThrLysLysAsnTyrSerLeuArgLysLeuLysThrGlyThrAla 20

v v v v
SerValAlaValAlaLeuThrValLeuGlyAlaGlyPheAlaAsnGlnThrThrValLys 40

A
v v v v
AlaGluGlyAlaLysIleAspTrpGlnGluGluTyrLysLysLeuAspGluAspAsnAla 60

v v v v
LysLeuValGluValValGluThrThrSerLeuGluAsnGluLysLeuLysSerGluAsn 80

v v v v
GluGluAsnLysLysAsnLeuAspLysLeuSerLysGluAsnGlnGlyLysLeuGluLys 100

v v v v
LeuGluLeuAspTyrLeuLysLysLeuAspHisGluHisLysGluHisGlnLysGluGln 120

B
v v v v
GlnGluGlnGluGluArgGlnLysAsnGlnGluGlnLeuGluArgLysTyrGlnArgGlu 140

C1
v v v v
ValGluLysArgTyrGlnGluGlnLeuGlnLysGlnGlnGlnLeuGluThrGluLysGln 160

v v v v
IleSerGluAlaSerArgLysSerLeuSerArgAspLeuGluAlaSerArgAlaAlaLys 180

C2
v v v v
LysAspLeuGluAlaGluHisGlnLysLeuGluAlaGluHisGlnLysLeuLysGluAsp 200

v v v v
LysGlnIleSerAspAlaSerArgGlnGlyLeuSerArgAspLeuGluAlaSerArgAla 220

v v v v
AlaLysLysGluLeuGluAlaAsnHisGlnLysLeuGluAlaGluHisGlnLysLeuLys 240

C3
v v v v
GluAspLysGlnIleSerAspAlaSerArgGlnGlyLeuSerArgAspLeuGluAlaSer 260

v v v v
ArgAlaAlaLysLysGluLeuGluAlaAsnHisGlnLysLeuGluAlaGluAlaLysAla 280
D
v v v v
LeuLysGluGlnLeuAlaLysGlnAlaGluGluLeuAlaLysLeuArgAlaGlyLysAla 300
v v v v
SerAspSerGlnThrProAspThrLysProGlyAsnLysAlaValProGlyLysGlyGln 320
v v v v
AlaProGlnAlaGlyThrLysProAsnGlnAsnLysAlaProMetLysGluThrLysArg 340
v
GlnLeuProSerThrGlyGluThrAlaAsnProPhePheThrAlaAlaAlaLeuThrVal 360
v v v v
MetAlaThrAlaGlyValAlaAlaValValLysArgLysGluGluAsn 376

2. Protein nach Anspruch 1, dadurch gekennzeichnet, daβ es aus der Aminosäurefrequenz von Aminosäure Nr. 1 bis Nr. 158 inklusive besteht, welches Protein Protein H' genannt wird.

3. DNA-Sequenz codierend für das Protein gemäβ einem der Ansprüche 1 oder 2, wie sie aus der Sequenz unten hergeleitet werden kann.

ATGACTAGACAACAAACCAAGAAAAATTATTCACTACGGAAACTAAAAACCGGTACGGCT 60
v v v v v v
TCAGTAGCCGTTGCTTTGACCGTTTTGGGCGCAGGTTTTGCAAACCAAACAACAGTTAAG 120
v v v v v v
GCGGAAGGGGCTAAAATTGATTGGCAAGAAGAGTATAAAAAGTTAGACGAAGATAATGCT 180
v v v v v v
AAACTTGTTGAGGTTGTTGAAACCACAAGTTTGGAAAACGAAAAACTCAAGAGTGAGAAT 240
v v v v v v
GAGGAGAATAAGAAAAATTTAGACAAACTTAGCAAAGAAAATCAAGGAAAGCTCGAAAAA 300
v v v v v v
TTGGAGCTTGACTATCTCAAAAAATTAGATCACGAGCACAAAGAGCACCAAAAAGAACAA 360
v v v v v v
CAAGAACAAGAAGAGCGACAAAAAAATCAAGAACAATTAGAACGTAAATACCAACGAGAA 420
v v v v v v
GTAGAAAAACGTTATCAAGAACAACTCCAAAAACAACAACAATTAGAAACAGAAAAGCAA 480
v v v v v v
ATCTCAGAAGCTAGTCGTAAGAGCCTAAGCCGTGACCTTGAAGCGTCTCGTGCAGCTAAA 540

v v v v v v
AAAGACCTTGAAGCTGAGCACCAAAAACTTGAAGCTGAGCACCAAAAACTTAAAGAAGAC 600

v v v v v v
AAACAAATCTCAGACGCAAGTCGTCAAGGCCTAAGCCGTGACCTTGAAGCGTCTCGTGCA 660

v v v v v v
GCTAAAAAAGAGCTTGAAGCAAATCACCAAAAACTTGAAGCTGAGCACCAAAAACTTAAA 720

v v v v v v
GAAGACAAACAAATCTCAGACGCAAGTCGTCAAGGCCTAAGCCGTGACCTTGAAGCGTCT 780

v v v v v v
CGTGCAGCTAAAAAAGAGCTTGAAGCAAATCACCAAAAACTTGAAGCAGAAGCAAAAGCA 840

v v v v v v
CTCAAAGAACAATTAGCGAAACAAGCTGAAGAACTTGCAAAACTAAGAGCTGGAAAAGCA 900

v v v v v v
TCAGACTCACAAACCCCTGATACAAAACCAGGAAACAAAGCTGTTCCAGGTAAAGGTCAA 960

v v v v v v
GCACCACAAGCAGGTACAAAACCTAACCAAAACAAAGCACCAATGAAGGAAACTAAGAGA 1020

v v v v v v
CAGTTACCATCAACAGGTGAAACAGCTAACCCATTCTTCACAGCGGCAGCCCTTACTGTT 1080

v v v v v
ATGGCAACAGCTGGAGTAGCAGCAGTTGTAAAACGCAAAGAAGAAAACTAA 1131

4. DNA-Sequenz, die unter konventionellen Bedingungen zu einer DNA-Sequenz gemäβ Anspruch 3 hybridisiert und ein Protein codiert, das die gleichen Bindungseigenschaften wie die Proteine nach einem der Ansprüche 1 oder 2 zeigt.

5. Rekombinanter Plasmidvektor, der eine DNA-Sequenz gemäβ einem der Ansprüche 3 oder 4 aufweist.

6. Wirtszelle, die mit dem Plasmid nach Anspruch 5 transformiert ist.

7. Wirtszelle gemäβ Anspruch 6, die zu der Spezies E.coli gehört.

8. Verfahren zur Herstellung einer Form eines Protein H gemäβ einem der Ansprüche 1 oder 2, das eine Kultivierung einer Wirtszelle gemäβ Anspruch 6 oder 7 unter geeigneten Bedingungen, ein Akkumulieren dieses Proteins in der Kultur oder die Lysierung der Zellen und seine Gewinnung davon aufweist.

9. Reagenziensatz zum Binden, Trennen und Identifizieren eines Humanimmunoglobulin G, dadurch gekennzeichnet, daβ es ein Protein gemäβ einem der Ansprüche 1 oder 2 aufweist.

10. Zusammensetzung, die ein Protein gemäβ einem der Ansprüche 1 oder 2 und fakultativ Additive oder Träger aufweist

11. Pharmazeutische Zusammensetzung, die ein Protein gemäβ einem der Ansprüche 1 oder 2 und fakultativ pharmazeutisch akzeptable Additive oder Träger aufweist.

## Revendications

1. Forme tronquée de protéine H, qui n'est pas susceptible de se fixer à l'albumine, caracterisée en ce que tout fragment de l'acide aminé numéro 159 à l'acide aminé numéro 376 de la séquence d'acides aminés de la protéine H indiquée ci-dessous est supprimé, et sous-fragments et variantes de la forme tronquée de protéine H qui ne fixent pas l'albumine, la séquence originale d'acides aminés de ces variantes étant modifiée ou altérée par insertion, par addition, par substitution, par inversion ou par suppression d'un acide aminé ou de plusieurs acides aminés.

v v v v
MetThrArgGlnGlnThrLysLysAsnTyrSerLeuArgLysLeuLysThrGlyThrAla 20

v v v v
SerValAlaValAlaLeuThrValLeuGlyAlaGlyPheAlaAsnGlnThrThrValLys 40

A
v v v v
Ala[GluGlyAlaLysIleAspTrpGlnGluGluTyrLysLysLeuAspGluAspAsnAla 60

v v v v
LysLeuValGluValValGluThrThrSerLeuGluAsnGluLysLeuLysSerGluAsn 80

v v v v
GluGluAsnLysLysAsnLeuAspLysLeuSerLysGluAsnGlnGlyLysLeuGluLys 100

v v v v
LeuGluLeuAspTyrLeuLysLysLeuAspHisGluHisLysGluHisGlnLysGluGln 120

B
v v v v
Gln[GluGlnGluGluArgGlnLysAsnGlnGluGlnLeuGluArgLysTyrGlnArgGlu 140

C1
v v v v
ValGluLysArgTyrGlnGluGlnLeuGlnLysGlnGlnLeuGluThrGlu[LysGln 160

IleSerGluAlaSerArgLysSerLeuSerArgAspLeuGluAlaSerArgAlaAlaLys 180
C2
LysAspLeuGluAlaGluHisGlnLysLeuGluAlaGluHisGlnLysLeuLysGluAsp 200
LysGlnIleSerAspAlaSerArgGlnGlyLeuSerArgAspLeuGluAlaSerArgAla 220
AlaLysLysGluLeuGluAlaAsnHisGlnLysLeuGluAlaGluHisGlnLysLeuLys 240
C3
GluAspLysGlnIleSerAspAlaSerArgGlnGlyLeuSerArgAspLeuGluAlaSer 260
ArgAlaAlaLysLysGluLeuGluAlaAsnHisGlnLysLeuGluAlaGluAlaLysAla 280
D
LeuLysGluGlnLeuAlaLysGlnAlaGluGluLeuAlaLysLeuArgAlaGlyLysAla 300
SerAspSerGlnThrProAspThrLysProGlyAsnLysAlaValProGlyLysGlyGln 320
AlaProGlnAlaGlyThrLysProAsnGlnAsnLysAlaProMetLysGluThrLysArg 340
GlnLeuProSerThrGlyGluThrAlaAsnProPhePheThrAlaAlaAlaLeuThrVal 360
MetAlaThrAlaGlyValAlaAlaValValLysArgLysGluGluAsn 376

2. Protéine suivant la revendication 1, caractérisée en ce qu'elle consiste en la séquence d'acides aminés allant de l'acide aminé numéro 1 à l'acide aminé numéro 158 inclus, cette protéine étant dénommée protéine H'.

3. Séquence d'ADN codant pour la protéine suivant l'une quelconque des revendications 1 ou 2, telle qu'elle peut être déduite de la séquence ci-dessous.

ATGACTAGACAACAAACCAAGAAAAATTATTCACTACGGAAACTAAAAACCGGTACGGCT 60
v v v v v v
TCAGTAGCCGTTGCTTTGACCGTTTTGGGCGCAGGTTTTGCAAACCAAACAACAGTTAAG 120
v v v v v v
GCGGAAGGGGCTAAAATTGATTGGCAAGAAGAGTATAAAAAGTTAGACGAAGATAATGCT 180
v v v v v v
AAACTTGTTGAGGTTGTTGAAACCACAAGTTTGGAAAACGAAAAACTCAAGAGTGAGAAT 240
v v v v v v
GAGGAGAATAAGAAAAATTTAGACAAACTTAGCAAAGAAAATCAAGGAAAGCTCGAAAAA 300

TTGGAGCTTGACTATCTCAAAAAATTAGATCACGAGCACAAAGAGCACCAAAAAGAACAA 360

CAAGAACAAGAAGAGCGACAAAAAAATCAAGAACAATTAGAACGTAAATACCAACGAGAA 420

GTAGAAAAACGTTATCAAGAACAACTCCAAAAACAACAACAATTAGAAACAGAAAAGCAA 480

ATCTCAGAAGCTAGTCGTAAGAGCCTAAGCCGTGACCTTGAAGCGTCTCGTGCAGCTAAA 540

AAAGACCTTGAAGCTGAGCACCAAAAACTTGAAGCTGAGCACCAAAAACTTAAAGAAGAC 600

AAACAAATCTCAGACGCAAGTCGTCAAGGCCTAAGCCGTGACCTTGAAGCGTCTCGTGCA 660

GCTAAAAAAGAGCTTGAAGCAAATCACCAAAAACTTGAAGCTGAGCACCAAAAACTTAAA 720

GAAGACAAACAAATCTCAGACGCAAGTCGTCAAGGCCTAAGCCGTGACCTTGAAGCGTCT 780

CGTGCAGCTAAAAAAGAGCTTGAAGCAAATCACCAAAAACTTGAAGCAGAAGCAAAAGCA 840

CTCAAAGAACAATTAGCGAAACAAGCTGAAGAACTTGCAAAACTAAGAGCTGGAAAAGCA 900

TCAGACTCACAAACCCCTGATACAAAACCAGGAAACAAAGCTGTTCCAGGTAAAGGTCAA 960

GCACCACAAGCAGGTACAAAACCTAACCAAAACAAAGCACCAATGAAGGAAACTAAGAGA 1020

CAGTTACCATCAACAGGTGAAACAGCTAACCCATTCTTCACAGCGGCAGCCCTTACTGTT 1080

ATGGCAACAGCTGGAGTAGCAGCAGTTGTAAAACGCAAAGAAGAAAACTAA 1131

4. Séquence d'ADN s'hybridant à une séquence d'ADN de la revendication 3, dans des conditions classiques et codant pour une protéine présentant les mêmes propriétés de fixation que les protéines suivant l'une quelconque des revendications 1 ou 2.

5. Vecteur de plasmide recombinant contenant une séquence d'ADN suivant l'une quelconque des revendications 3 ou 4.

6. Cellule hôte transfannée par le plasmide recombinant de la revendication 5.

7. Cellule hôte suivant la revendication 6, qui appartient à l'espace E. coli.

8. Procédé de préparation d'une forme de protéine H suivant l'une des revendications 1 ou 2, qui consiste à cultiver une cellule hôte suivant la revendication 6 ou 7 dans des conditions convenables, à accumuler la protéine dans la culture ou à lyser les cellules et a recueillir la protéine à partir de la culture ou des cellules.

9. Trousse de réactifs pour la fixation, la séparation et l'identification de l'immunoglobuline G humaine, caractérisée en ce qu'elle comprend une protéine suivant l'une quelconque des revendications 1 ou 2.

10. Composition comprenant une protéine suivant l'une quelconque des revendications 1 ou 2 et éventuellement des additifs ou des supports.

11. Composition pharmaceutique comprenant une protéine suivant l'une quelconque des revendications 1 ou 2 et éventuellement des additifs ou des supports acceptables pharmaceutiquement.
